# EUROPEAN PATENT SPECIFICATION

(11) **EP 2 642 933 B1**
(45) Date of publication and mention of the grant of the patent: **13.01.2016**
(21) Application number: 10803139.4
(22) Date of filing: 23.11.2010
(51) Int. Cl.: A61B 17/70

(54) **INTERSPINOUS VERTEBRAL DISTRACTOR**
INTERSPINALER WIRBELDISTRAKTOR
DISTRACTEUR VERTÉBRAL INTER-ÉPINEUX

(43) Date of publication of application: 02.10.2013
(73) Proprietor: Calvosa, Giuseppe, 56123 Pisa (IT)
(72) Inventor: BARTALESI, Raphael, I-50122 Firenze (FI) (IT); TENUCCI, Miria, I-55100 Lucca (LU) (IT)
(74) Representative: Papa, Elisabetta
(86) International application number: PCT/IB2010/055379
(87) International publication number: WO 2012/069878

(56) References cited:
- WO-A-2007/075788
- WO-A-2009/098536
- US-A1- 2006 264 938
- US-A1- 2007 032 790
- US-A1- 2007 225 807
- US-A1- 2008 108 990

## Description

The present invention refers to an intervertebral distractor of interspinous type suitable for a percutaneous insertion, and in particular to a distractor of the type comprising a double pair of lateral stabilizers, applied on a main body and preferably selectively spreadable apart with respect to the latter to hold the body itself in position between two adjacent spinous processes.

Intervertebral distractors are devices apt to space apart two adjacent vertebrae. In particular, the distractors subject-matter of the present invention are prostheses conceived to be permanently implanted in the space comprised between the spinous processes of adjacent vertebrae, in order to maintain an intervertebral distraction such as to limit the loads transmitted between said vertebrae by effect, e.g., of degenerative pathologies of the intervertebral discs, and to contain the associated painful manifestations.

With respect to other vertebral prostheses, the interspinous distractors can be easily inserted in their seat, thanks to the relative ease with which the spinous processes of two adjacent vertebrae allow slight spreading apart. For the same reason, such distractors do not compromise local mobility of the rachis in flexion, but reduce hyperextension thereof.

WO 2009/098536 discloses an interspinous distractor of the aforementioned type.

Against said advantages, however, known stabilization problems subsist. In fact, the distractor has to be maintained in position, in particular has to be constrained with respect to displacements such as to compromise its functionality or even cause its ejection from the seat, with movements in the frontal plane of the patient. Such a stabilization function is generally carried out by lateral fins of the distractor, apt to abut on the spinous apophyses.

However, such fins risk damaging the surrounding bone and joint tissues, especially in the presence of specific pathologies.

Therefore, the technical problem set and solved by the present invention is that of providing an intervertebral distractor of the above-mentioned type, allowing to overcome the drawbacks mentioned above with reference to the known art.

Such a problem is solved by an intervertebral distractor according to claim 1.

Preferred features of the present invention are set forth in the dependent claims thereof.

In the present context - and coherently with the current anatomical terminology - by "*frontal plane"* (or "coronal plane") is meant a plane which runs parallel to the forehead (or to the coronal suture) of the subject.

The present invention provides some relevant advantages. One of the main advantages lies in the fact that in the distractor of the invention the tilt of the stabilizers with respect to the frontal plane, i.e. the deployment thereof not on an exactly vertical plane but on an oblique plane, allows a moving away from the anatomical frontal plane where there are, to the right and left of the spinous processes, the corresponding articular processes of the vertebral level concerned.

The moving away of the lateral stabilizers of the distractor of the invention from the articular saliences (prominences) is particularly advantageous in case of degenerative pathologies in which the articular processes are arthrosic and hypertrophic.

The distractor of the invention therefore allows a movement among the muscles of the stabilizers, to overcome any conflict with the articular bone plane.

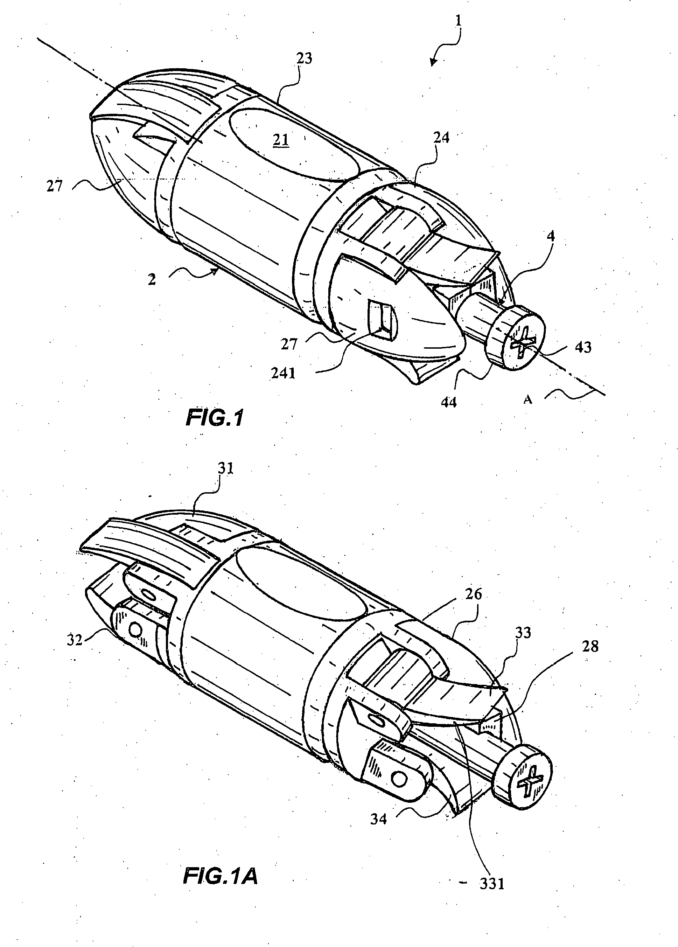

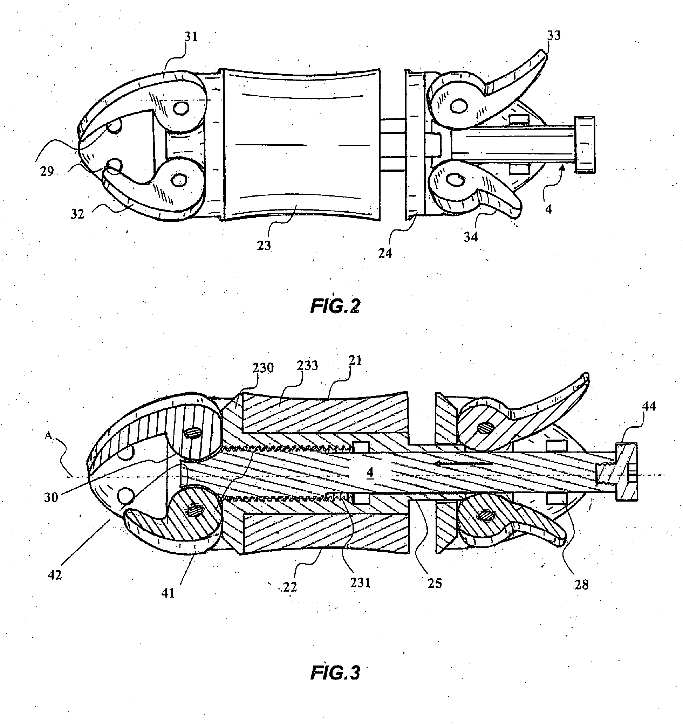

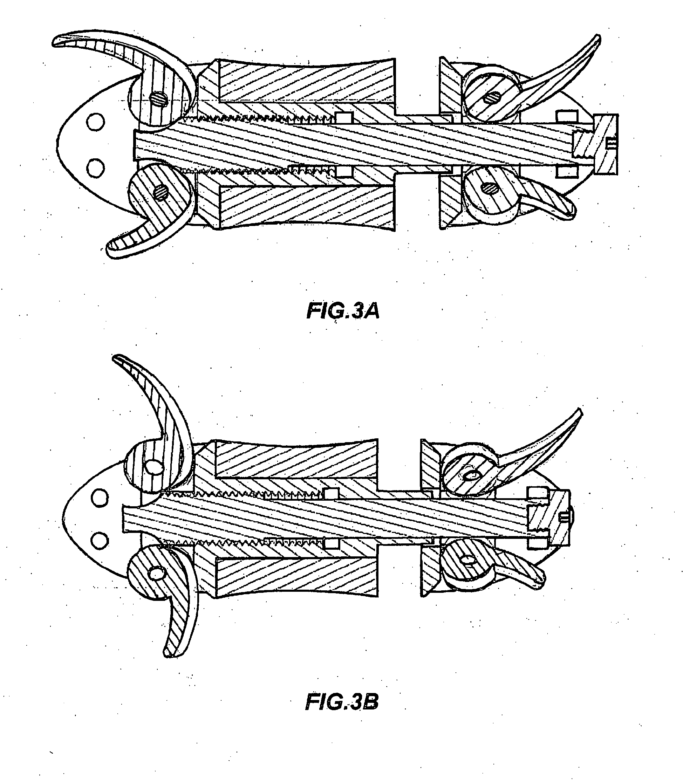

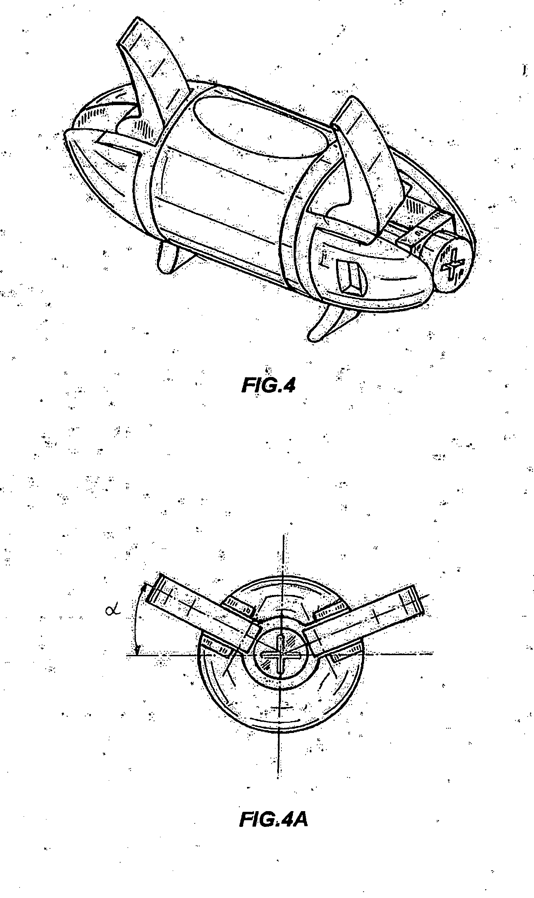

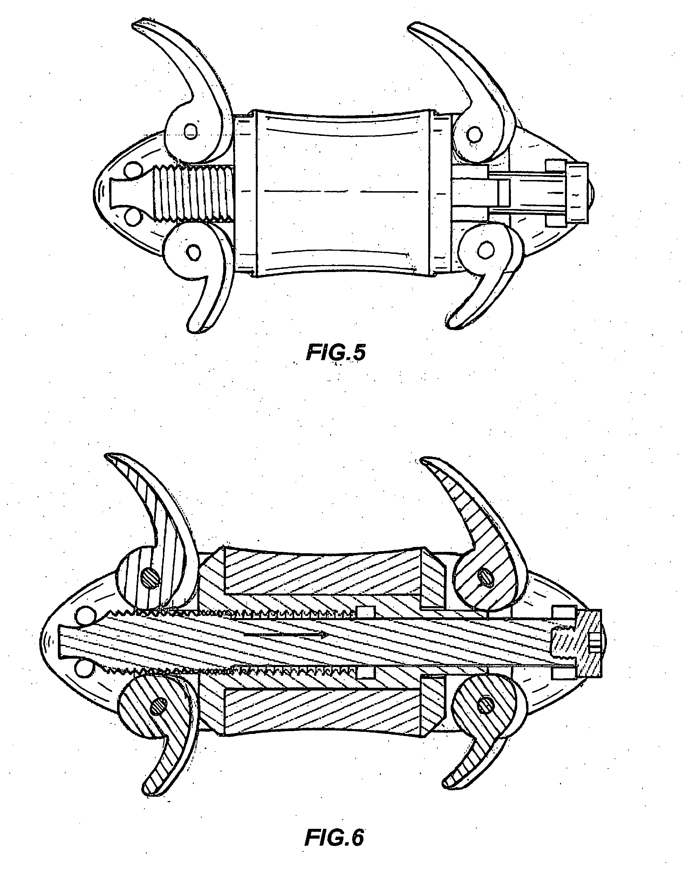

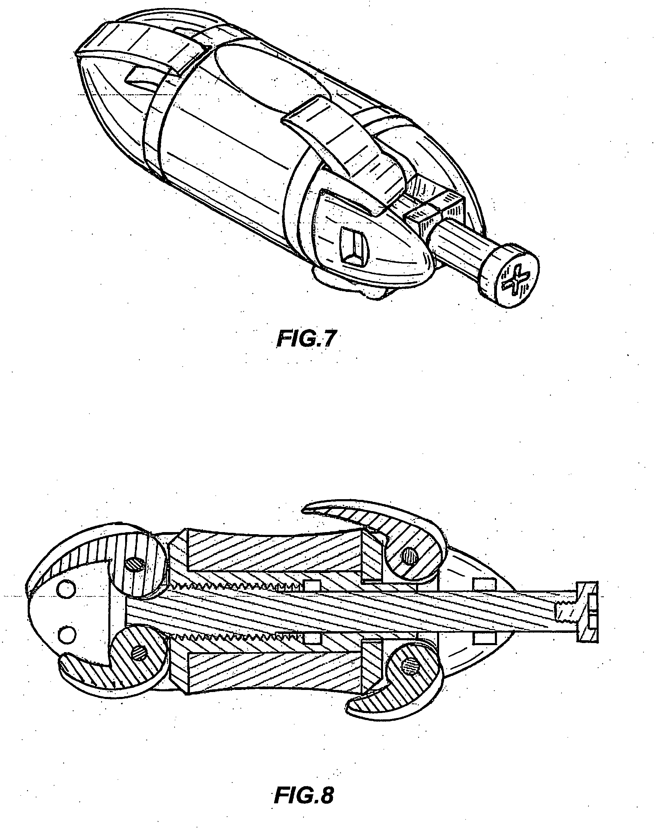

Other advantages, features, and the operation steps of the present invention will be made apparent in the following detailed description of some embodiments thereof, given by way of example and not for limitative purposes. Reference will be made to the figures of the annexed drawings, wherein:
▪ Figure 1 shows a perspective view of a preferred embodiment of the intervertebral distractor of the invention, in a first inserting configuration for insertion into a patient's body, in which the lateral stabilizers are closed;
▪ Figure 1A shows a perspective view of the distractor of Figure 1, in which two lateral protective shells have been removed for greater clarity;
▪ Figure 2 shows a front view of the distractor of Figure 1, again with two lateral shells removed;
▪ Figure 3 shows a longitudinal sectional view of the distractor of Figure 1;
▪ Figures 3A and 3B show each another longitudinal sectional view of the distractor of Figure 1, in a respective intermediate configuration for opening the distal stabilizers with respect to the surgeon;
▪ Figure 4 shows a perspective view of the distractor of Figure 1, in a second configuration for holding *in situ* into a patient's body, in which the lateral stabilizers are spread apart;
▪ Figure 4A shows a side view of the distractor of Figure 4, from the distal side thereof;
▪ Figure 5 shows a front view of the distractor of Figure 1, in the configuration of Figure 4, with two lateral shells removed;
▪ Figure 6 shows a longitudinal sectional view of the distractor of Figure 1, in the configuration of Figure 4;
▪ Figure 7 shows a perspective view of a variant embodiment of the distractor of the invention, in a further extracting configuration in which the lateral stabilizers arranged proximally with respect to the surgeon's position are further spread apart, and those arranged distally are closed; and
▪ Figure 8 shows a longitudinal sectional view of the distractor of Figure 7.

Referring initially to Figures 1, 1A, 2 and 3, an intervertebral distractor of interspinous type according to a preferred embodiment of the invention is generally denoted by 1.

The distractor 1 has a generally tapered configuration, fostering a percutaneous insertion thereof.

The distractor 1 comprises first of all a main body 2, apt to be inserted between two adjacent spinous processes to provide a corresponding support. In the present embodiment, the body 2 has a substantially elongated configuration with generally substantially elliptic cross sections. The body 2 bears, topwise and bottomwise, a respective depression or concavity 21, 22, substantially forming a saddle, apt to foster its own stabilization *in situ* between two spinous processes. It will be understood that in the present context the definitions of "top" and "bottom" are referred to the position of the distractor 1 when used *in situ* with the subject in standing position.

The main body 2 has a longitudinal axis denoted by A.

The main body 2 is implemented in two portions, respectively 23 and 24, slidably coupled to each other according to modes that will be detailed hereinafter. For greater clarity, such two portions 23 and 24 will be denoted respectively as first and second portion, or respectively as distal and proximal portion. These latter two terms are to be understood, here and hereinafter, as referred to the position of the distractor 1 with respect to the surgeon during insertion into a patient's body.

The first portion 23, of greater extension, has a tubular prolongation, or appendage 25, with a substantially cylindrical development, expending symmetrically along the longitudinal axis A and engaging a corresponding seat of the second portion 24.

As will be illustrated hereinafter, such tubular appendage 25 implements an abutment means against the means for spreading apart (retractors) that will be described hereinafter.

To the second portion 24 of the body 2 it is also integrally associated a substantially C-shaped member 28, implementing a means for stopping a rod of the distractor, rod which will it also be introduced hereinafter.

Moreover, the main body 2 bears, in correspondence of or near to a first longitudinal end thereof associated to the above-mentioned first portion 23, two pairs of flanges 26 for coupling with lateral stabilizers that will be described below. An analogous double pair of flanges 26 is provided in correspondence of or near to the second longitudinal end of the body 2 associated to the second portion 24 thereof.

Moreover, in correspondence of each of said first and second end, the main body 2 has a pair of protective shells transversally placed side by side, denoted by 27, which have a configuration tapered in the direction of the longitudinal axis A, thereby fostering the percutaneous insertion of the distractor 1.

The first portion 23 of the body 2 bears also a pair of pins 29 integral to one of said shells 27 and internal projecting therefrom, the function of which will be made clear hereinafter.

As seen, e.g., in Figure 2, in the present embodiment the first portion 23 is in turn formed by a frame 230 bearing the above-mentioned appendage 25, the shells 27, the pins 29 and the flanges 26, and by a distractor body 233 bearing the saddles 21 and 22, arranged externally to the frame 230 itself and integral therewith. Preferably, the frame 230 is made of Titanium, whereas the distractor body 233 is made of PEEK.

The distractor 1 further comprises a first - distal - pair of stabilizers, denoted by 31 and 32, and a second - proximal - pair of stabilizers, denoted by 33 e 34, associated respectively to said first and second portion 23, 24 of the distractor 1. In particular, each stabilizer 31-34 is rotatably connected to the respective portion 23, 24 of distractor in correspondence of a flange 26 thereof. More specifically, each stabilizer 31-34 is hinged on said flange in correspondence of an end thereof.

Each stabilizer 31-34 has a substantially elongated, curved fin-like configuration, with a first convex profile and a corresponding concave profile. By way of example, the convex profile of the proximal stabilizer 33 is denoted by 331, and the concave one by 332.

The rotatable connection with the main body 2 enables the stabilizers 31-34 of each pair to rotate between a first closed position, shown in Figure 1, in which they implement a minimal encumbrance configuration, fostering a percutaneous insertion of the distractor 1 into a patient's body, and a second opened configuration, shown in Figure 4, in which they are spread apart so as to be able to engage from a respective side the spinous apophyses, holding *in situ* the main body 2.

As shown in Figures 1, 1A, 2 and 3, the overall arrangement is such that in the closed configuration the stabilizers 31 and 32 of the first pair have their own concave profile facing towards the main body 2, i.e. giving onto axis A, and the stabilizers 33 and 34 of the second pair have instead their own convex profile in such position.

On the contrary, in the spread apart configuration of Figures 4, 5 and 6, the stabilizers 33 and 34 of the second pair have, in general, their own concave profile facing towards the main body 2, i.e. giving onto axis A.

As shown in the drawings, preferably the bottom stabilizers 32 and 34 of the two pairs have an extension generally lower than the corresponding top stabilizers 31 and 33 of the same pair. This contrivance allows to prevent interferences of the distractor 1 with the laminae and/or the articular facets of the lower vertebra concerned by the distractor itself. This is particularly useful in case of bone hypertrophies or deformations, scoliosis, and in the case of vertebral levels comprised in the range L5-S1.

As best seen in Figure 4A, according to the invention each stabilizer has a tilt with respect to the frontal plane. By way of example, in Figure 4A such tilt is denoted by α for the stabilizer 31.

Preferably, for each stabilizer 31-34 the above-mentioned tilt is comprised in a range of about 8-35 degrees. In an even more preferred embodiment, such tilt is equal to about 30 degrees.

While in the present embodiment all four stabilizers 31-34 provided the above-mentioned tilt with respect to the frontal plane, variant embodiments may provide a subgroup, and at least only one of the stabilizers present, to have the above-mentioned tilt; this always in order to locally solve the technical problem set forth in the introduction.

Turning to the embodiment disclosed in the figures described hereto, the distractor 1 further comprises an elongated element, or rod, generally denoted by 4, percutaneously actuatable to cause the passage of the stabilizers 31-34 from the first to the second configuration illustrated above.

Such rod 4 is housed within the main body 2, and in particular within the frame 230, in correspondence of the longitudinal axis A and slidably coupled to such body 2, so that the related movement be carried out precisely along such axis A. In particular, the rod 4 extends within the first and the second portion 23 and 24 of the body 2, engaging also the above-mentioned tubular appendage 25 of the first portion 23.

In the present example, the slidable coupling is of screw-nut screw threaded type, the rod 4 bearing, in correspondence of its own distal portion, a threading 41 complementary to a corresponding nut screw threading 231 obtained internally to the first portion 23.

The rod 4 further has, in correspondence of a distal end thereof, spreading means for spreading apart the stabilizers 31 and 32 of the first pair, which in the present embodiment are implemented by a shaped profile 42 apt to form a shape coupling with such stabilizers 31 and 32 so as to cause precisely, when needed, the spreading apart.

Always in the present example, the shaped profile 42 of the rod 4 is of cam type. In particular, the shaped profile 42 is of concave type and the stabilizers 31 and 32 of the first pair have, in correspondence of the rotatable connection to the main body 2, a corresponding convex profile 30 conjugated with the concave profile 42.

Moreover, the rod 4 has, substantially oppositely to the shaped profile 42, means for coupling with a percutaneously actuatable manipulation instrument. In the present example, such means is implemented by a further shaped profile 43, obtained on a head 44 and apt to be engaged by a screwdriver instrument. To a technician in the field it will be obvious that the end 44 may have other forms of coupling for the actuation instrument.

The rod 4 may also be made hollow in order to allow, e.g., the insertion of a guide wire or the like.

The above-mentioned member 28 also increases the rigidity of the rod 4, limiting its flexure.

Hereinafter, the operation of the distractor 1 will be described with regard to the passage of the stabilizers 31-34 from the first to the second position illustrated above.

At the percutaneous insertion of the distractor 1, the latter appears in closed configuration, as shown in Figures 1, 1A, 2 and 3. The stabilizers are therefore rotated into a minimal encumbrance position, with their own convex/concave profiles arranged as already described above.

The rod 4 appears in a position maximally set back in the proximal direction. In particular, its concave profile 42 engages the complementary convex profile 30 of the stabilizers 31 and 32 of the first pair, so that said stabilizers may assume the above-mentioned closed position. In the maximally set back position, the concave profile 42 is such as to oppose to an accidental opening up of the stabilizers 31 and 32, opening up that the tissues might cause during an insertion of the device.

The percutaneous insertion of the distractor can occur by cannula and/or guide wires according to procedures already known to the technician in the field, or to innovative procedures subject of a separate patent application.

During such insertion, the main body 2 can be held by engagement of a dedicated instrument into suitable lateral seats of the second portion 24, one of which is exemplarily denoted by 241 in Figure 1. Concomitantly, the advancement of the distractor may be obtained by acting with a conventional instrument on the head 44 of the rod 4.

Once completed the actual inserting step, the main body 2 is housed between two adjacent spinous processes. Then, the rod 4 is percutaneously actuated by a screwdriver instrument in order to produce the selective spreading apart of the stabilizers 31-34, and this according to the procedure illustrated hereinafter.

First of all, the rod 4 is slid along the longitudinal axis A of the main body 2 in a distal direction, as per arrow reported in Figure 3. In such movement, the rod slides with respect to both portions 23 and 24 of the main body 2, which therefore remains stationary. Following such sliding, the concave profile 42 of the rod 4 engages the corresponding convex profile 30 of the latter stabilizers 31 and 32 so as to cause their spreading apart, as shown in Figure 3A.

The further sliding therefore causes the full spreading apart of the stabilizers 31 and 32, shown in Figures 4 to 6. In such a spread apart configuration, such stabilizers are externally abutted on the lateral spinous apophyses, preventing movement of the distractor 1 in the proximal direction. As shown in Figures 5 and 6, further distal movement of the rod 4 is prevented, always in the configuration considered herein, by the engagement of the concave profile 42 thereof with the pins 29, implementing therefore means for the distal stopping of the rod itself.

Always in the fully spread apart configuration of the stabilizers 31 and 32, the head 44 of the rod 4 is abutted against the stopping means 28 associated to the second portion 24 of the body 2. Such means 28 is therefore it also apt to cause the stopping of the distal movement of the rod 4.

In the configuration presently considered, a further rotation of the screwdriver instrument engaging the rod 4 causes a reversion of the related motion, in the sense of producing the sliding in the proximal direction of the first portion 23 of the main body 2 with respect to the rod 4 itself and to the second portion 24, as indicated by the arrow reported in Figure 6. Therefore, such proximal sliding causes the approaching of the tubular appendage 25 to the stabilizers 33 and 34 of the second pair, the subsequent abutting of the former on corresponding convex profiles of the latter, and then the spreading apart thereof shown in Figures 4 to 6.

In the completely spread apart position, the first and the second portion 23 and 24 of the main body are abutted against each other, forming one body.

Upon reaching such spreading apart, the stabilizers 33 and 34 of the second pair are abutted on the spinous apophyses contralateral with respect to those of the first pair 31, 32.

Incidentally, it has to be noted that during the insertion of the distractor and the opening up of the distal stabilizers 31 and 32 it is not possible to accidentally open the proximal ones 33 and 34, as these are retained by the cannulation. Only when the distal stabilizers 31 and 32 have opened up, the cannula is slightly unthreaded and the opening up of the proximal stabilizers is allowed.

By now, it will be better appreciated that the rod 4 allows a reversible spreading apart of the stabilizers 31-34, in the sense of allowing, by reversing the hereto-described procedure, the reclosing thereof.

It will also be appreciated that the arrangement described allows an independent spreading of the stabilizers of the first pair with respect to those of the second pair (and vice versa).

Moreover, it should be noted that the arrangement described allows a continuous change of the position of the stabilizers of the two pairs.

It will also be appreciated that a single instrument, in the case considered herein a screwdriver, is required to perform the spreading apart of both pairs of stabilizers.

Furthermore, the technician in the field will appreciate that it is possible to provide, in combination with the hereto-described components of the distractor, also specific means for holding one or more stabilizers in the above-described extreme spread apart and closed positions, means that can be disengaged when needed.

Referring to Figures 7 and 8, on the basis of a variant embodiment it is provided that the stabilizers 33 and 34 of the second pair may rotate with respect to the main body 2 of an angle greater than about 90 degrees, and in particular preferably of an angle comprised in a range of about 120-180 degrees.

Such increased angle is advantageous since it is possible, when it is necessary to extract the distractor 1, to bring said stabilizers 33 and 34 into a further minimal encumbrance configuration in which they are substantially "upturned" on the distractor body 233 and expose their convex profile so as to facilitate the extraction itself.

Therefore, overall the stabilizers of the first pair may be continuously rotated among three reference positions; specifically, a closed position of insertion, a spread apart position of holding *in situ* and a further spread apart extracting position. The stabilizers of the first pair can be rotated between the first two positions mentioned above.

The present invention has been hereto described with reference to preferred embodiments thereof. It is understood that other embodiments might exist, all falling within the concept of the same invention, as defined by the protective scope of the claims hereinafter.

## Claims

1. An intervertebral distractor (1) of interspinous type, comprising:
- a distraction main body (2), apt to be inserted between two adjacent spinous processes to provide a corresponding support; and
- a first (31, 32) and a second (33, 34) pair of lateral stabilizers, connected to said main body (2) and arranged respectively in correspondence of or near to a first and a second end of said main body (2), the stabilizers of each pair having or being able to assume a spread apart configuration projecting with respect to said main body (2) so as to be able to engage from a respective side the spinous apophyses, holding in position said main body (2),
**characterized in that**, in said projecting configuration, said stabilizers of said first (31, 32) and second (33, 34) pair have a tilt (α) with respect to the frontal plane, by deploying on an oblique plane and not on a vertical plane, so that they are moved away from the anatomical frontal plane where there are the corresponding articular processes of the vertebral level concerned.

2. The intervertebral distractor (1) according to claim 1, wherein said tilt (α) is comprised in a range of about 8-35 degrees.

3. The intervertebral distractor (1) according to the preceding claim, wherein said tilt (α) is comprised in a range of about 10-30 degrees.

4. The intervertebral distractor (1) according to claim 2 or 3, wherein said tilt (α) is equal to about 30 degrees.

5. The intervertebral distractor (1) according to any one of the preceding claims, wherein each stabilizer of said pairs is movable with respect to said main body (2) between a closed position, in which the stabilizers of the pair implement a minimal encumbrance configuration, fostering a percutaneous insertion of the distractor into the patient's body, and said projecting spread apart configuration.

6. The intervertebral distractor (1) according to the preceding claim, wherein the stabilizers (31, 32) of said first pair have each a concave profile that is facing, in said closed position, towards said main body (2).

7. The intervertebral distractor (1) according to claim 5 or 6, wherein the stabilizers (33, 34) of said second pair have each a convex profile that is facing, in said closed position, towards said main body (2).

8. The intervertebral distractor (1) according to any one of the claims 5 to 7, wherein each stabilizer of said pairs is rotatably connected to said main body (2).

9. The intervertebral distractor (1) according to the preceding claim, wherein the stabilizers (31-34) of said pairs are hinged on said main body (2) in correspondence of respective ends.

10. The intervertebral distractor (1) according to any one of the claims 5 to 9, wherein the stabilizers (33, 34) of said second pair are apt to assume a third, further spread apart, extracting position.

11. The intervertebral distractor (1) according to the preceding claim, wherein the stabilizers (33, 34) of said second pair are rotatably connected to said main body (2) and apt to rotate with respect to said main body (2) of an angle greater than about 90 degrees, so as to take a further minimal encumbrance configuration.

12. The intervertebral distractor (1) according to the preceding claim, wherein said angle of rotation is comprised in a range of about 120-180 degrees.

13. The intervertebral distractor (1) according to any one of the claims 5 to 12, comprising actuation means (4) percutaneously actuatable and apt to cause, when required, the spreading apart of the stabilizers (31, 32) of said first and/or second pair.

14. The intervertebral distractor (1) according to the preceding claim, wherein the overall arrangement is such as to cause a continuous change of the position of the stabilizers (31-34) of said pairs.

15. The intervertebral distractor (1) according to claims 13 or 14, wherein the overall arrangement is such as to cause an independent spreading apart of the stabilizers respectively of said first (31, 32) and second (33, 34) pair.

16. The intervertebral distractor (1) according to any one of the claims 13 a 15, wherein said actuation means is percutaneously actuatable and have spreading means (42) apt to cause, when required, a spreading apart of the stabilizers (31, 32) of said first pair.

17. The intervertebral distractor (1) according to the preceding claim, wherein said spreading means comprises a shaped profile (42) apt to form a shape coupling with the stabilizers (31, 32) of said first pair.

18. The intervertebral distractor (1) according to the preceding claim, wherein said shaped profile (42) is of cam type.

19. The intervertebral distractor (1) according to claim 17 or 18, wherein said shaped profile (42) is of concave type and the stabilizers (31, 32) of said first pair have a corresponding convex profile (30) conjugated with said concave profile.

20. The intervertebral distractor (1) according to any one of the claims 13 to 19, wherein said main body (2) comprises a first (23) and a second (24) portion slidably coupled and bearing respectively said first (31, 32) and second (33, 34) pair of stabilizers, the overall arrangement being such that the actuation of said actuating means (4) causes a relative sliding between said first (23) and second (24) portion which in turn produces the spreading apart of the stabilizers (33, 34) of said second pair.

21. The intervertebral distractor (1) according to any one of the claims 13 to 20, wherein said actuation means comprises an elongated element (4) slidably coupled with said main body (2).

22. The intervertebral distractor (1) according to the preceding claim, wherein said elongated element (4) is movable within said main body (2) along a longitudinal axis (A) of the latter.

23. The intervertebral distractor (1) according to any one of the preceding claims, wherein a stabilizer (32, 34) of each of said pairs has an extension lower than the other stabilizer (31, 33) of the same pair.

## Patentansprüche

1. Zwischenwirbeldistraktor (1) vom interspinalen Typ, der umfasst:
- einen Distraktionshauptkörper (2), der geeignet ist zwischen zwei angrenzenden Dornfortsätzen eingesetzt zu werden, um eine entsprechende Unterstützung zur Verfügung zu stellen; und
- ein erstes (31, 32) und ein zweites (33, 34) Paar von seitlichen Stabilisatoren, die mit dem Hauptkörper (2) verbunden sind und in Übereinstimmung mit oder nahe zu einem ersten beziehungsweise einem zweiten Ende des Hauptkörpers (2) angeordnet sind, wobei die Stabilisatoren eines jeden Paares über eine auseinander gespreizte Konfiguration verfügen, oder diese annehmen können, die bezüglich des Hauptkörpers (2) vorspringen, so dass sie in der Lage sind von einer entsprechenden Seite mit den spinalen Apophysen eine Verbindung einzugehen, wodurch sie den Hauptkörper (2) in Position halten können,
**dadurch gekennzeichnet, dass**, in der vorspringenden Konfiguration, die Stabilisatoren des ersten (31, 32) und zweiten (33, 34) Paares über einen Neigungswinkel (α) bezüglich der Frontalebene verfügen, durch Bereitstellung auf einer schrägen Ebene und nicht auf einer vertikalen Ebene, so dass sie von der anatomischen Frontalebene weg bewegt werden, wo sich die entsprechenden Gelenkfortsätze des betreffenden Wirbelsäulenbereichs befinden.

2. Zwischenwirbeldistraktor (1) gemäß Anspruch 1, wobei der Neigungswinkel (α) innerhalb eines Bereichs von ungefähr 8 - 35 Grad liegt.

3. Zwischenwirbeldistraktor (1) gemäß dem vorangehenden Anspruch, wobei der Neigungswinkel (α) innerhalb eines Bereichs von ungefähr 10 - 30 Grad liegt.

4. Zwischenwirbeldistraktor (1) gemäß Anspruch 2 oder 3, wobei der Neigungswinkel (α) ungefähr 30 Grad entspricht.

5. Zwischenwirbeldistraktor (1) gemäß einem der vorangehenden Ansprüche, wobei jeder Stabilisator der Paare bezüglich des Hauptkörpers (2) zwischen einer geschlossenen Stellung, in der die Stabilisatoren des Paares eine Konfiguration mit minimaler Belastung implementieren, die eine perkutane Einsetzung des Distraktors in den Körper des Patienten begünstigt, und der vorspringenden auseinander gespreizten Konfiguration beweglich ist.

6. Zwischenwirbeldistraktor (1) gemäß dem vorangehenden Anspruch, wobei die Stabilisatoren (31, 32) des ersten Paares beide über ein konkaves Profil verfügen, das, in der geschlossenen Stellung, dem Hauptkörper (2) gegenüberliegend angeordnet ist.

7. Zwischenwirbeldistraktor (1) gemäß Anspruch 5 oder 6, wobei die Stabilisatoren (33, 34) des zweiten Paares beide über ein konvexes Profil verfügen, das, in der geschlossenen Stellung, dem Hauptkörper (2) gegenüberliegend angeordnet ist.

8. Zwischenwirbeldistraktor (1) gemäß einem der Ansprüche 5 bis 7, wobei jeder Stabilisator der Paare mit dem Hauptkörper (2) drehbar verbunden ist.

9. Zwischenwirbeldistraktor (1) gemäß dem vorangehenden Anspruch, wobei die Stabilisatoren (31 - 34) der Paare auf dem Hauptkörper (2) bezüglich der jeweiligen Enden klappbar sind.

10. Zwischenwirbeldistraktor (1) gemäß einem der Ansprüche 5 bis 9, wobei die Stabilisatoren (33, 34) des zweiten Paares geeignet sind eine dritte, weiter auseinander gespreizte, extrahierende Stellung einzunehmen.

11. Zwischenwirbeldistraktor (1) gemäß dem vorangehenden Anspruch, wobei die Stabilisatoren (33, 34) des zweiten Paares drehbar mit dem Hauptkörper (2) verbunden sind und geeignet sind sich bezüglich des Hauptkörpers (2) um einen Winkel zu drehen, der größer als ungefähr 90 Grad ist, um so eine weitere minimale Belastungskonfiguration einzunehmen.

12. Zwischenwirbeldistraktor (1) gemäß dem vorangehenden Anspruch, wobei der Rotationswinkel in einem Bereich von ungefähr 120 - 180 Grad liegt.

13. Zwischenwirbeldistraktor (1) gemäß einem der Ansprüche 5 bis 12, der umfasst: das Betätigungsmittel (4), das perkutan betätigt werden kann und geeignet ist, wenn erforderlich, das Auseinanderspreizen der Stabilisatoren (31, 32) des ersten und/oder zweiten Paares zu bewirken.

14. Zwischenwirbeldistraktor (1) gemäß dem vorangehenden Anspruch, wobei die Gesamtanordnung so ist, dass sie eine kontinuierliche Änderung der Stellung der Stabilisatoren (31 - 34) der Paare bewirken kann.

15. Zwischenwirbeldistraktor (1) gemäß den Ansprüchen 13 oder 14, wobei die Gesamtanordnung so ist, dass sie ein unabhängiges Auseinanderspreizen der Stabilisatoren des ersten (31, 32) beziehungsweise des zweiten (33, 34) Paares bewirken kann.

16. Zwischenwirbeldistraktor (1) gemäß einem der Ansprüche 13 bis 15, wobei das Betätigungsmittel perkutan betätigt werden kann und über das Spreizmittel (42) verfügt, das, wenn erforderlich, ein Auseinanderspreizen der Stabilisatoren (31, 32) des ersten Paares bewirken kann.

17. Zwischenwirbeldistraktor (1) gemäß dem vorangehenden Anspruch, wobei das Spreizmittel ein geformtes Profil (42) umfasst, das geeignet ist eine Form zu bilden, die mit den Stabilisatoren (31, 32) des ersten Paares koppelt.

18. Zwischenwirbeldistraktor (1) gemäß dem vorangehenden Anspruch, wobei das geformte Profil (42) nockenartig ist.

19. Zwischenwirbeldistraktor (1) gemäß Anspruch 17 oder 18, wobei das geformte Profil (42) von konkaver Art ist und die Stabilisatoren (31, 32) des ersten Paares über ein entsprechendes konvexes Profil (30) verfügen, das mit dem konkaven Profil konjugiert.

20. Zwischenwirbeldistraktor (1) gemäß einem der Ansprüche 13 bis 19, wobei der Hauptkörper (2) einen ersten (23) und einen zweiten (24) Teil umfasst, die verschiebbar gekoppelt sind und jeweils das erste (31, 32) und zweite (33, 34) Paar von Stabilisatoren tragen, wobei die Gesamtanordnung so ist, dass die Betätigung des Betätigungsmittels (4) ein relatives Gleiten zwischen dem ersten (23) und zweiten (24) Teil erzeugt, was wiederum das Auseinanderspreizen der Stabilisatoren (33, 34) des zweiten Paares bewirkt.

21. Zwischenwirbeldistraktor (1) gemäß einem der Ansprüche 13 bis 20, wobei das Betätigungsmittel ein verlängertes Element (4) umfasst, das verschiebbar mit dem Hauptkörper (2) gekoppelt ist.

22. Zwischenwirbeldistraktor (1) gemäß dem vorangehenden Anspruch, wobei das verlängerte Element (4) in dem Hauptkörper (2) entlang einer Längsachse (A) des letzteren beweglich ist.

23. Zwischenwirbeldistraktor (1) gemäß einem der vorangehenden Ansprüche, wobei ein Stabilisator (32, 34) eines jeden der Paare über eine Verlängerung verfügt, die geringer als die des anderen Stabilisators (31, 33) des selben Paares ist.

## Revendications

1. Distracteur intervertébral (1) du type inter-épineux, comprenant :
- un corps principal de distraction (2), apte à être inséré entre deux apophyses épineuses adjacentes pour fournir un support correspondant ; et
- une première (31, 32) et une seconde (33, 34) paire de stabilisateurs latéraux, raccordées audit corps principal (2) et agencées respectivement en correspondance de ou à proximité d'une première et d'une seconde extrémité dudit corps principal (2), les stabilisateurs de chaque paire ayant ou pouvant adopter une configuration écartée faisant saillie par rapport audit corps principal (2) afin de pouvoir se mettre en prise à partir d'un côté respectif des apophyses épineuses, maintenant en position dudit corps principal (2),
**caractérisé en ce que**, dans ladite configuration en saillie, lesdits stabilisateurs desdites première (31, 32) et seconde (33, 34) paires ont une inclinaison (α) par rapport au plan frontal, en se déployant sur un plan oblique et pas sur un plan vertical, de sorte qu'ils sont éloignés du plan frontal anatomique où l'on trouve les apophyses articulaires correspondantes du niveau vertébral concerné.

2. Distracteur intervertébral (1) selon la revendication 1, dans lequel ladite inclinaison (α) est comprise dans une plage d'environ 8-35 degrés.

3. Distracteur intervertébral (1) selon la revendication précédente, dans lequel ladite inclinaison (α) est comprise dans une plage d'environ 10-30 degrés.

4. Distracteur intervertébral (1) selon la revendication 2 ou 3, dans lequel ladite inclinaison (α) est égale à environ 30 degrés.

5. Distracteur intervertébral (1) selon l'une quelconque des revendications précédentes, dans lequel chaque stabilisateur desdites paires est mobile par rapport audit corps principal (2) entre une position fermée, dans laquelle les stabilisateurs de la paire mettent en oeuvre une configuration d'encombrement minimum, accueillant une insertion percutanée du distracteur dans le corps du patient, et ladite configuration écartée en saillie.

6. Distracteur intervertébral (1) selon la revendication précédente, dans lequel les stabilisateurs (31, 32) de ladite première paire ont chacun un profil concave qui est orienté, dans ladite position fermée, vers ledit corps principal (2).

7. Distracteur intervertébral (1) selon la revendication 5 ou 6, dans lequel les stabilisateurs (33, 34) de ladite seconde paire ont chacun un profil convexe qui est orienté, dans ladite position fermée, vers ledit corps principal (2).

8. Distracteur intervertébral (1) selon l'une quelconque des revendications 5 à 7, dans lequel chaque stabilisateur desdites paires est raccordé en rotation audit corps principal (2).

9. Distracteur intervertébral (1) selon la revendication précédente, dans lequel les stabilisateurs (31-34) desdites paires sont articulés sur ledit corps principal (2) en correspondance des extrémités respectives.

10. Distracteur intervertébral (1) selon l'une quelconque des revendications 5 à 9, dans lequel les stabilisateurs (33, 34) de ladite seconde paire sont aptes à adopter une troisième position d'extraction davantage écartée.

11. Distracteur intervertébral (1) selon la revendication précédente, dans lequel les stabilisateurs (33, 34) de ladite seconde paire sont raccordés en rotation audit corps principal (2) et aptes à tourner par rapport audit corps principal (2) selon un angle supérieur à environ 90 degrés, afin de prendre une configuration d'encombrement minimum supplémentaire.

12. Distracteur intervertébral (1) selon la revendication précédente, dans lequel ledit angle de rotation est compris dans une plage d'environ 120-180 degrés.

13. Distracteur intervertébral (1) selon l'une quelconque des revendications 5 à 12, comprenant des moyens d'actionnement (4) pouvant être actionnés par voie percutanée et aptes à provoquer, lorsque cela est nécessaire, l'écartement des stabilisateurs (31, 32) desdites première et/ou seconde paires.

14. Distracteur intervertébral (1) selon la revendication précédente, dans lequel l'agencement global est tel qu'il provoque un changement continu de la position des stabilisateurs (31-34) desdites paires.

15. Distracteur intervertébral (1) selon les revendications 13 ou 14, dans lequel l'agencement global est tel qu'il provoque l'écartement indépendant des stabilisateurs respectivement desdites première (31, 32) et seconde (33, 34) paires.

16. Distracteur intervertébral (1) selon l'une quelconque des revendications 13 à 15, dans lequel lesdits moyens d'actionnement peuvent être actionnés, par voie percutanée et ont des moyens d'écartement (42) aptes à provoquer, lorsque cela est nécessaire, un écartement des stabilisateurs (31, 32) de ladite première paire.

17. Distracteur intervertébral (1) selon la revendication précédente, dans lequel lesdits moyens d'écartement comprennent un profil formé (42) apte à former un couplage de forme avec les stabilisateurs (31, 32) de ladite première paire.

18. Distracteur intervertébral (1) selon la revendication précédente, dans lequel ledit profil formé (42) est du type à came.

19. Distracteur intervertébral (1) selon la revendication 17 ou 18, dans lequel ledit profil formé (42) est du type concave et les stabilisateurs (31, 32) de ladite première paire ont un profil convexe (30) correspondant conjugué avec ledit profil concave.

20. Distracteur intervertébral (1) selon l'une quelconque des revendications 13 à 19, dans lequel ledit corps principal (2) comprend une première (23) et une seconde (24) partie couplée de manière coulissante et supportant respectivement lesdites première (31, 32) et seconde (33, 34) paires de stabilisateurs, l'agencement global étant tel que l'actionnement desdits moyens d'actionnement (4) provoque un coulissement relatif entre lesdites première (23) et seconde (24) parties, qui produit à son tour l'écartement des stabilisateurs (33, 34) de ladite seconde paire.

21. Distracteur intervertébral (1) selon l'une quelconque des revendications 13 à 20, dans lequel lesdits moyens d'actionnement comprennent un élément allongé (4) couplé de manière coulissante audit corps principal (2).

22. Distracteur intervertébral (1) selon la revendication précédente, dans lequel ledit élément allongé (4) est mobile à l'intérieur dudit corps principal (2) le long d'un axe longitudinal (A) de ce dernier.

23. Distracteur intervertébral (1) selon l'une quelconque des revendications précédentes, dans lequel un stabilisateur (32, 34) de chacune desdites paires a une extension inférieure à l'autre stabilisateur (31, 33) de la même paire.
